# EUROPEAN PATENT APPLICATION

(11) **EP 3 417 903 A1**
(43) Date of publication of application: **26.12.2018**
(21) Application number: 18275082.8
(22) Date of filing: 15.06.2018
(51) Int. Cl.: A61M 25/10, A61F 2/954, A61F 2/958

(54) **OFFSET BALLOONS FOR LUMEN SUPPORT OR DILATION**

(30) Priority: 23.06.2017 US 201715631797
(71) Applicant: Cook Medical Technologies LLC, Bloomington, IN 47404 (US)
(72) Inventor: Eaton, Elizabeth A., Bloomington, IN 47401 (US)
(74) Representative: Williams Powell

(57) **Abstract**

A system for providing dilation and support to a body vessel includes a shaft having two compliant balloons (318,319) attached to the shaft and spaced apart longitudinally from each and defining a longitudinal intermediate space between them. The shaft includes an inflation lumen for inflating the balloons (318,319) and a delivery lumen for delivering secondary balloons (350) into the intermediate space. Secondary balloons (350) are deliverable over wires (340) that extend through the delivery lumen and out of the side-ports of the shaft, with the secondary balloons (350) delivered through the side ports and to a desired location and inflated in the intermediate space or adjacent the intermediate space, such as to branch vessels. The compliant balloons are inflatable toward each other to take up the intermediate and conform to the shape of the body vessel and the secondary balloons.

## Description

### TECHNICAL FIELD

This invention relates to endoluminal medical devices for introduction into the human or animal body for treatment of endovascular disease.

### BACKGROUND OF THE INVENTION

The functional vessels of human and animal bodies, such as blood vessels and ducts, occasionally weaken or even rupture. For example, the aortic wall can weaken, resulting in an aneurysm, or it may develop a tear in one of the layers of the aortic wall resulting in an aortic dissection.

One common surgical intervention for weakened, aneurysmal or ruptured passageways or ducts involves the introduction of a compliant balloon into the damaged blood vessel.

One type of surgical intervention utilizing the insertion of a balloon into the patient's vasculature is Percutaneous Transluminal Angioplasty (PTA), often referred to simply as angioplasty, for opening up a blocked blood vessel. This procedures involves the insertion of a balloon catheter through the vasculature and to the desired location of the blockage. The balloon is inflated and deflated at the location of the blockage, thereby opening up the blood vessel.

Another type of surgical intervention involving balloons is a procedure where a balloon catheter is introduced toward a blood vessel, such as the aorta, to repair a dissection that has occurred. In this procedure, a compliant balloon is introduced to a location adjacent the tear in the vessel wall, and the balloon is inflated to block blood flow through the "true" lumen of the blood vessel, allowing the filling/thrombosis of the "false" lumen.

In many cases, however, the damaged or defected portion of the vasculature may include a branch vessel branching from the main vessel or may be near one of these branch vessels. For example, in the case of the abdominal aorta, there are at least three major branch vessels, including the celiac, mesenteric, and renal arteries, as well as other vessels, leading to various other body organs.

Thus, in the case of a vessel blockage, it can be difficult to open up the blockage near the branch vessel or in the branch vessel itself with a traditional balloon, and it may be undesirable to inflate a balloon across an opening of a branch vessel to repair a blockage in a main vessel. In the case of a vessel dissection, inflating the balloon across a branch vessel opening may not effectively block the true lumen.

### SUMMARY OF THE INVENTION

The present invention seeks to provide an improved medical system and method.

According to an aspect of the present invention, there is provided a medical system as specified in claim 1.

In particular, a system is provided having a balloon catheter including a shaft and a first balloon attached to the shaft and a second balloon attached to the shaft. The first and second balloons are spaced apart longitudinally on the shaft. At least one inflation lumen extends within the shaft and is in fluid communication an interior cavity of the first balloon and an interior cavity of the second balloon.

The first balloon and the second balloon define an intermediate space longitudinally between the first balloon and the second balloon outside of the shaft. At least one delivery lumen extends longitudinally through the shaft and is in fluid communication with the intermediate space via a side-port or hole of the shaft. The delivery lumen is configured to deliver medical devices through the shaft and into the intermediate space through the side-port. At least one secondary balloon is moveable through the at least one delivery lumen and out of the side-port into the intermediate space. The first and second balloons are inflatable to take up the intermediate space.

The system may include two inflation lumens, one for each of the balloons, where a first inflation lumen is in communication with the interior cavity of the first balloon and a second inflation lumen is in communication with the interior cavity of the second balloon. In this approach, the balloons are independently inflatable and deflatable. In another approach, there is a single inflation lumen that is in communication with the interior cavities of both balloons that controls the inflation of both balloons.

The system may include multiple delivery lumens instead of a single delivery lumen. The multiple delivery lumens will each have a corresponding side-port or hole that communicates with the intermediate space between the balloons. The side ports may be disposed at different longitudinal or circumferential locations on the shaft. The side ports may each be disposed longitudinally between the first and second balloons.

They system may include wires that can be pre-loaded through the delivery lumen or lumens that extend through the side ports and provide a delivery path for the secondary balloons to be delivered through the delivery lumen or lumens.

The first and second balloons may be compliant balloons, and the secondary balloons may be minimally compliant balloons, such that the first and second balloons, when inflated, will conform to the shape of the minimally compliant balloons.

In another example, a medical system includes a balloon catheter including a shaft and a first balloon attached to the shaft and a second balloon attached to the shaft. The first and second balloons are spaced apart longitudinally on the shaft. At least one inflation lumen extends within the shaft and is in fluid communication an interior cavity of the first balloon and an interior cavity of the second balloon.

The first balloon and the second balloon define an intermediate space longitudinally between the first balloon and the second balloon outside of the shaft. At least one delivery lumen extends longitudinally through the shaft and is in fluid communication with the intermediate space via a side-port or hole of the shaft. The delivery lumen is configured to deliver medical devices through the shaft and into the intermediate space through the side-port. At least one secondary balloon is moveable through the at least one delivery lumen and out of the side-port into the intermediate space. At least one wire extends through the delivery, with the secondary balloon deliverable over the wire.

The system includes a delivery configuration and a deployed configuration. In the delivery configuration, the first and second balloons are in a compressed configuration. In the deployed configuration, the first and second balloons are in an expanded configuration.

In the delivery configuration, the secondary balloons do not extend beyond the side-port of the shaft. In the deployed configuration, the secondary balloons are disposed at least partially through the side-port.

In the delivery configuration, the secondary balloon may be disposed outside of the shaft. In the deployed configuration when the first and second balloons are inflated, the first and second balloons may expand longitudinally toward each other and occupy the intermediate space. Further, in the deployed configuration, the secondary balloons may be disposed in the intermediate space. In another approach, one or more of the secondary balloons may extend beyond the intermediate space in the deployed configuration.

In the delivery configuration, the wires may extend through the delivery lumen and out of the side-port into the intermediate space. Alternatively, in the delivery configuration, the wires may terminate prior to the side-port.

The first and second balloons may be compliant balloons and the secondary balloons may be minimally compliant, where the first and second balloons conform to the shape of the body vessel and the shape of the secondary balloon when inflated.

In another example, there is described a method for delivering a system for providing dilation and support to a body vessel includes delivering, to a body vessel, a shaft having a first inflatable balloon and a second inflatable balloon attached to the shaft and spaced apart longitudinally on the shaft. The first and second balloons are inflatable via at least one inflation lumen that extends through the shaft. The inflation lumen is in fluid communication with the first and second inflatable balloons.

The shaft further includes at least one delivery lumen extending through the shaft and including at least one side-port in fluid communication with an intermediate space disposed longitudinally between the first and second balloons.

The method further includes inflating the first and second balloons into engagement with a wall of the body vessel and delivering at least one inflatable secondary balloon through the at least one delivery lumen out of the side-port and into the intermediate space. The method also includes inflating the secondary balloon.

The first and second balloons are compliant and the secondary balloon is minimally compliant. The first and second balloons inflate into the intermediate space around the secondary balloon and conform to the shape of the body vessel and the secondary balloon.

The shaft may include at least one wire extending through the delivery lumen when the shaft is delivered. The method may include delivering the wire into a branch vessel prior to inflating the first and second balloons.

In one approach, the secondary balloons are delivered over the wire and into the branch vessel prior to inflating the balloons. In another approach, the secondary balloons are delivered after the first and second balloons are inflated.

The shaft may include multiple delivery lumens rather than a single delivery lumen, and the secondary balloons may be delivered through different delivery lumens and over wires that extend through the different delivery lumens.

The method may also include deflating the first and second balloons after delivering the secondary balloons and re-inflating the balloons after inflating the secondary balloons.

The foregoing paragraphs have been provided by way of general introduction, and are not intended to limit the scope of the following claims. The presently preferred embodiments, together with further advantages, will be best understood by reference to the following detailed description taken in conjunction with the accompanying drawings.

### BRIEF DESCRIPTION OF THE DRAWINGS

Embodiments of the present invention are described below, by way of example only, with reference to the accompanying drawings, in which:
Figure 1 shows a balloon catheter having an inflatable balloon having holes formed in an outer wall, and a shaft having a delivery lumen in communication with the interior of the balloon, and wires extending out of the delivery lumen and through the interior of the balloon and out of the holes;
Figure 2A is a cross-sectional view of one embodiment of the shaft, showing a delivery lumen, an inflation lumen for the balloon, and a wire lumen for delivering the balloon catheter over a guidewire;
Figure 2B is another embodiment, where the shaft includes a delivery lumen and a guidewire lumen;
Figure 2C is a schematic cross-sectional view illustrating a tube that extends from the insertion end of the shaft that is integral with the shaft, with the guidewire lumen extending through the tube;
Figure 3 is a schematic view of a daughter balloon having a shaft and an inflation lumen;
Figure 4 is a cross-sectional view of the shaft of the daughter balloon;
Figure 5 is a schematic view of the daughter balloon delivered over the wire through the delivery lumen and into the balloon and out of the hole in the balloon, and inflated into engagement with the hole;
Figure 5A illustrates a reinforcing band extending around the hole in the balloon;
Figure 5B illustrates a mesh material embedded in the balloon wall around the hole in the balloon;
Figure 6 is a view of multiple daughter balloons delivered into the holes of the balloon, and the balloon inflated after each of the daughter balloons have been delivered and inflated;
Figure 7 is a cross-sectional view of the balloon catheter and the balloon in a compressed state within a delivery sheath, with wires preloaded in a delivery state;
Figure 8 illustrates the balloon catheter delivered to a body vessel and exposed from the delivery sheath, with the wires being routed into adjacent branch vessels from the holes in the balloon;
Figure 9 illustrates the daughter balloons delivered across the holes and inflated into engagement with the holes and extending into the branch vessels;
Figure 10 illustrates the balloon in an inflated condition and expanded into contact with the body vessel wall with the daughter balloons inflated into engagement with the branch vessels;
Figure 11 illustrates another embodiment of a balloon catheter having a shaft and first and second balloons attached to the shaft and spaced apart longitudinally from each other, with the shaft including side-ports or holes that are open to an intermediate space between the first and second balloons, and wires extending out of the side-ports;
Figure 12 illustrates a cross-sectional view of the shaft showing a single inflation lumen that communicates with both balloons, a wire lumen for delivering the catheter over the wire, and multiple delivery lumens for the wires and for delivering secondary balloons through the side-ports in the shaft;
Figures 13 and 13A illustrates another embodiment of the shaft showing two inflation lumens, one for each balloon, a wire lumen, and a single delivery lumen for the wires and for delivering secondary balloons to the side-ports in the shaft;
Figure 14 illustrates the balloon catheter of Figure 12 delivered to a body vessel, with the wires extended into adjacent branch vessels and the balloons inflated and expanded to take up the intermediate space;
Figure 15 illustrates the secondary balloons in a delivered state and extending partially into the branch vessels over the wires; and
Figure 16 illustrates the secondary balloons in a different delivered state, with one of the secondary balloons delivered fully out of the intermediate space between the balloons and into a branch vessel, and the other secondary balloon delivered into the intermediate space short of the branch vessel.

### DETAILED DESCRIPTION

Unless defined otherwise, all technical and scientific terms used herein have the same meaning as commonly understood to one of ordinary skill in the art to which this invention belongs.

The term "distal" means a location or direction that is, or a portion of a device that when implanted is further downstream in the direction of or with respect to blood flow. In the case of aortic intervention, distal means a location further away from the heart. In a trans-femoral approach, the distal end of a device is the end that is closer to the operator.

The term "proximal" means a location or direction that is, or a portion of a device that when implanted is further upstream in the direction of or with respect to blood flow. In the case of aortic intervention, proximal means a location closer to the heart. In a trans-femoral approach, the proximal end of a device is the insertion end of the device.

The term "fenestration" means an opening provided through a surface of a prosthesis from the interior of the prosthesis to the exterior of the prostheses and may have a variety of geometries, including circular, semi-circular, oval, oblong, as well as other geometries.

The term "biocompatible" refers to a material that is substantially non-toxic in the in vivo environment of its intended use, and that is not substantially rejected by the patient's physiological system (i.e., is non-antigenic). Examples of biocompatible materials from which textile graft material can be formed include, without limitation, polyesters, such as polyethylene terephthalate; fluorinated polymers, such as polytetrafluoroethylene (PTFE) and fibers of expanded PTFE, and polyurethanes. In addition, materials that are not inherently biocompatible may be subjected to surface modifications in order to render the materials biocompatible. Examples of surface modifications include graft polymerization of biocompatible polymers on the materials surface, coating of the surface with a crosslinked biocompatible polymer, chemical modification with biocompatible functional groups, and immobilization of a compatibilizing agent such as heparin or other biocompatible substances. Thus, any fibrous material having sufficient strength to survive in the in vivo environment may be used to form a textile graft, provided the final textile is biocompatible. Fibers suitable for making textile grafts include polyethylene, polypropylene, polyaramids, polyacrylonitrile, nylon, and cellulose, in addition to the polyesters, fluorinated polymers, and polyurethanes as listed above. Furthermore, bioremodelable materials may also be used singly or in combination with the aforementioned polymer materials. The textile may be made of one or more polymers that do not require treatment or modification to be biocompatible. The graft may be constructed from woven multifilament polyester, for example and without limitation, Dacron™, produced by DuPont. Dacron™ is known to be sufficiently biologically inert, non-biodegradable, and durable to permit safe insertion inside the human body.

The term "prosthesis" means any device for insertion or implantation into or replacement for a body part or function of that body part. It may also mean a device that enhances or adds functionality to a physiological system. The term prosthesis may include, for example and without limitation, a stent, stent-graft, filter, valve, balloon, embolization coil, and the like.

The term "tubular" refers to the general shape of an endoluminal device which allows the module to carry fluid along a distance or fit within a tubular structure such as an artery. Tubular prosthetic devices include single, branched, and bifurcated devices. Tubular may refer to any shape including, but not limited to, tapered, cylindrical, curvilinear, or any combination thereof. A tubular device may have a cross-sectional shape that is, circular, substantially circular or the like. However, it should be understood that the cross-sectional shape is not limited thereto, and other shapes, such as, for example, hexagonal, pentagonal, octagonal, or the like are contemplated. The term "endoluminal" refers to or describes objects that can be placed inside a lumen or a body passageway in a human or animal body. A lumen or a body passageway can be an existing lumen or a lumen created by surgical intervention. As used in this specification, the terms "lumen" or "body passageway" are intended to have a broad meaning and encompasses any duct (e.g., natural or iatrogenic) within the human body and can include a member selected from the group comprising: blood vessels, respiratory ducts, gastrointestinal ducts, and the like. "Endoluminal device" or "endoluminal prosthesis" thus describes devices that can be placed inside one of these lumens.

The term "branch vessel" refers to a vessel that branches off from a main vessel. Examples are the celiac and renal arteries which are branch vessels to the aorta (i.e., the main vessel in this context). As another example, the hypogastric artery is a branch vessel to the common iliac, which is a main vessel in this context. Thus, it should be seen that "branch vessel" and "main vessel" are relative terms.

"Longitudinally" refers to a direction, position or length substantially parallel with a longitudinal axis of a reference, and is the length-wise component of the helical orientation.

"Circumferentially" refers to a direction, position, or length that encircles a longitudinal axis of reference. The term "circumferential" is not restricted to a full 360° circumferential turn or to a constant radius.

The terms "patient," "subject," and "recipient" as used in this application refer to any animal, especially humans.

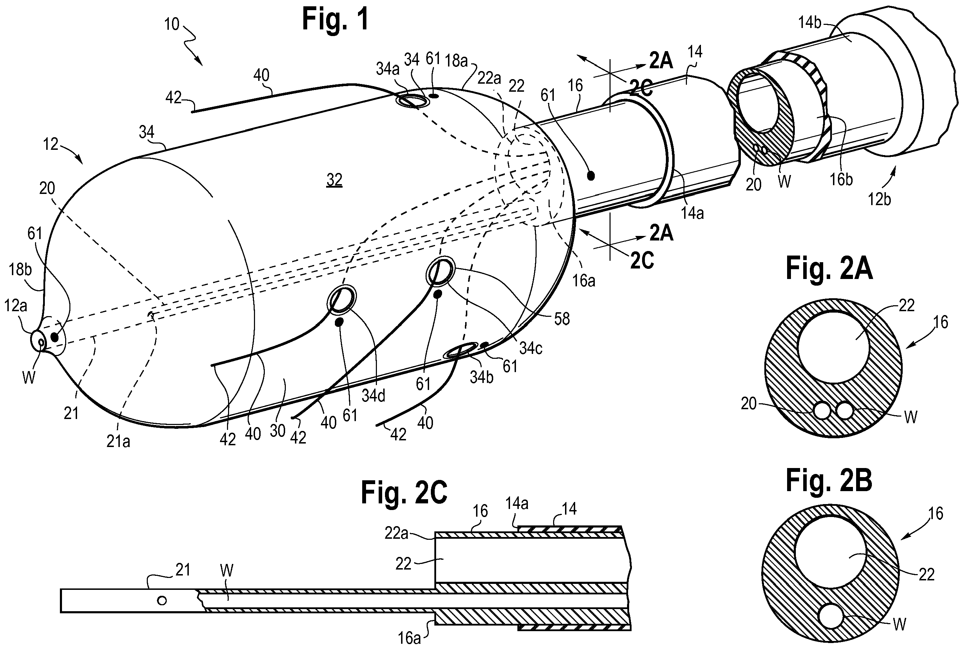

Figures 1-10 show a system 10 including a balloon catheter 12 and a delivery sheath 14 for delivering and deploying the balloon catheter 12 within a patient's vasculature at a desired location. As used herein, references to an insertion end refer to the end of a device or component that is inserted first into the patient and that is opposite an operator end, which is the end that typically remains out of the body.

Figure 1 is a schematic illustration of a balloon 18 in an expanded or partially expanded state that includes holes 34 allowing for additional balloons to pass through at least partially through a wall 30 of the balloon 18. These additional balloons are not shown in Figure 1 in order to illustrate the holes 34 and other structure. Figure 1 therefore illustrates the balloon 18 in an expanded condition, but in use, the balloon 18 typically remains in a compressed position prior to the additional balloons being delivered through the holes 34 to seal the holes 34. However, in some approaches, the balloon 18 may be expanded into a partially expanded state to aid in the introduction of the further balloons, as further described below.

The delivery sheath 14 includes an insertion end 14a and an operator end 14b. The balloon catheter 12 likewise includes an insertion end 12a and an operator end 12b.

The balloon catheter 12 includes a main tubular shaft 16 that extends longitudinally between an insertion end 16a and an operator end 16b, and an inflatable balloon 18 attached to the shaft 16 near the insertion end 16a of the shaft 16. As shown in Figure 1, the insertion end 12a of the overall balloon catheter 12 is disposed further away from the operator than the insertion end 16a of the shaft 16.

The shaft 16 defines an inflation lumen 20 that is in fluid communication with an interior cavity of the balloon 18. In one approach, the inflation lumen 20 may be defined by the shaft 16 and a tube 21 that extends from the insertion end 16a of the shaft 16 and into the interior of the balloon 18 and to the insertion end 12a of the balloon catheter 12. The shaft 16 and the tube 21 may define an inflation port 21 a that delivers inflation fluid from the inflation lumen 20 into the interior of the balloon 18, as shown in Figure 1. In one approach, the tube 21 may be a separate component that attaches to the end of the shaft 16, as illustrated in Figure 1. Alternatively, as shown in Figure 2C, the tube 21 may be an integral extension of the shaft 16. In one approach, as shown in Figure 2C, the tube 21 may provide support for the balloon catheter 12, and may include the guidewire lumen W, but where inflation fluid is provided through another lumen such as a delivery lumen 22, as described below.

The shaft 16 further defines a delivery lumen 22 extending longitudinally through the shaft 16 and configured to allow other system components to be housed therein or delivered therethrough. The catheter 12 and shaft 16 may be delivered with or without the use of a guidewire. In one another approach, the balloon catheter 12 may include a guidewire lumen W formed in the shaft 16 and extending through the tube 21 or other similar support structure that extends through the balloon 18, as shown in Figures 1-3. It will be appreciated that the various lumens can be arranged in a variety of ways in the shaft 16 and the through the balloon 18, such that the delivery lumen 22 opens into the interior of the balloon 18 and the inflation lumen 20 can provide inflation fluid to the interior of the balloon, with the guidewire lumen W extending through the balloon 18 and isolated from the inflation lumen 20. One example of a lumen arrangement is shown in Figure 2A, which shows a cross-section of the shaft 16 and the lumens extending therethrough, including guidewire lumen W, delivery lumen 22 and inflation lumen 20.

Figure 2B illustrates an embodiment where the shaft 16 includes the delivery lumen 22 and a guidewire lumen W, but without a separate inflation lumen. In this embodiment, inflation fluid may be delivered through the delivery lumen 22. The guidewire lumen W extends through the tube 21 in this embodiment, as shown in Figure 2C.

In one embodiment, the delivery lumen 22 is sized to be larger/wider than the inflation lumen 20. In particular, the delivery lumen 22 is sized to be wide enough to facilitate delivery of additional balloons through the delivery lumen 22, as well as multiple guidewires for each of the additional balloons, as further described below.

The balloon 18, attached to the insertion end 12a of the catheter 12 as well as to the insertion end 16a of the shaft 16, is preferably in the form of a compliant balloon, meaning that the balloon 18 will typically take the shape of the vessel in which it is deployed once inflated. The balloon 18 is preferably sized to correspond generally to the size of the vessel to which the balloon 18 will be delivered and inflated. The balloon 18 being in the form of a compliant balloon allows the balloon 18 to be inflated to occlude or fill a target blood vessel while limiting instances where the balloon 18 may cause further damage to the vessel wall when inflated. The compliant balloon 18, when inflated, will tend to take the shape of the blood vessel due to its compliant structure. The compliant, or semi-compliant in another approach, balloon 18 helps the balloon 18 accommodate variation in the vascular anatomy that may vary from patient to patient.

In the case of a traditional inflatable balloon, the balloon wall is typically intact such that the balloon will retain the inflation fluid that is introduced into the cavity defined by the balloon to inflate the balloon.

As shown in Figure 1, the balloon 18 includes the wall 30 that defines an interior cavity 32 therein. The interior cavity 32 is in fluid communication with the inflation lumen 20 of the catheter 12 and the delivery lumen 22 of the shaft 16, such that inflation fluid can be introduced into the interior cavity 32 via the inflation lumen 20 or the delivery lumen 22 to inflate and expand the balloon wall 30 in a manner known in the art, as well as allowing further medical devices to be introduced into the interior cavity 32 via the delivery lumen 22.

Furthermore, the balloon 18 defines one or more holes 34, or punctures or passageways or the like, in the wall 30 that permit the passage of additional structure through the wall 30. Accordingly, with the holes 34 extending through the wall 30, the balloon 18 differs from a traditional balloon in that inflation fluid introduced in a balloon with holes would leak out of the balloon absent other structure that will seal the holes. In the present approach, such structure is provided in the form of additional balloons, which are further described below. In one approach, the balloon wall 30 may include a reinforcing band 58, further described below, that surrounds each of the holes 34 to provide reinforcement to the holes 34 in response to additional balloons being inserted through the holes 34.

In one approach, the holes 34 are generally small. Exemplary holes may be about 2-4 mm in width. The size of the holes 34 are preferably selected to be smaller than the size of the ultimate structure that will be extended through the hole 34, such that after the structure is extended through the hole 34 and left in place, the holes 34 will be generally sealed due to the larger size of the inserted structure exerting a radially outward force on the holes 34, such that the interior cavity 32 within the balloon 18 may still be inflated in response to the introduction of inflation fluid. Accordingly, in one approach, 2-4 mm sized holes are one preferred sizing to accommodate a further balloon that inflates to about 8 mm in width, for example.

In one approach, the balloon 18 may include four holes 34a, 34b, 34c, and 34d. The holes 34a-d are located on the balloon 18 such that their location will typically correspond to the general location of branch vessels in the target delivery and deployment area. For example, the four-hole arrangement may be used in the abdominal aorta near the left and right renal arteries (LRA and RRA) and the supermesenteric artery (SMA) and celiac artery (CA). The SMA and CA are typically disposed above a patient's renal arteries, such that they are between a patient's renal arteries and the heart.

Thus, for a balloon 18 that is designed and arranged to be delivered to this area of the patient, holes 34a and 34b can be arranged on laterally opposite sides of the balloon 18 to accommodate the LRA and RRA, with holes 34c and 34d being disposed longitudinally offset from the holes 34a and 34b and generally on the same lateral side of the balloon 18 as each other. Differing anatomy may result in altering the arrangement of the holes 34 as needed. Further, depending on the desired location for introduction and inflation of the balloon 18, additional holes or fewer holes may be used.

The balloon 18 defines a first end 18a and a second end 18b. The first end 18a is preferably attached to the insertion end 16a of the shaft 16, with the second end 18b being attached to the insertion end 12a of the balloon catheter 12 at the opposite longitudinal end of the balloon 18 opposite the interface between the insertion end 16a of the shaft 16 and the first end 18a of the balloon 18. The delivery lumen 22 includes an opening 22a at the insertion end 16a of the shaft 16 that is in fluid communication with the interior cavity 32 of the balloon, such that wires or other structure can be passed through the delivery lumen 22 and into the interior cavity 32. The delivery lumen 22 therefore could be used for providing inflation fluid as an alternative to the inflation lumen 20 and tube 21, with the tube 21 being used for support rather than inflation, as described above, and the tube 21 may include the guidewire lumen W but remain fluidly isolated from the cavity 32 of the balloon 18, as shown in Figure 1B.

As shown in Figure 1, the system 10 may further include one or more wires 40 for assisting in the delivery of additional structure to the holes 34. The wires 40 will act as guidewires for the additional structure to allow for the additional structure to be routed to the desired hole 34. The number of wires 40 preferably corresponds to the number of holes 34 in the balloon 18. However, it will be appreciated that the number of wires 40 could differ from the number of holes 34 in some cases. Typically, each hole 34 will have a corresponding wire 40 extending through the hole 34.

Thus, in one approach, the wires 40 extend through the delivery lumen 22 and out of the insertion end 16a of the shaft 16 and into the interior cavity 32 defined by the balloon 18. Each individual wire 40 may further extend through a corresponding hole 34 of the balloon 18 and out of the interior cavity 32 of the balloon 18, such that a terminal end 42 of the wire 40 is disposed outside of the interior cavity 32. The wires 40 are preferably arranged in a pre-loaded state, such that they extend though the holes 34 of the balloon while the balloon 18 is housed within the delivery sheath 14 prior to insertion into the body. The wires 40 may be preloaded as packaged and provided to the doctor in a pre-loaded state, or the wires 40 may be loaded by the doctor prior to delivery of the catheter 12 into the patient. In either case, the wires 40 are preloaded in the catheter 12 in a delivery configuration prior to insertion into the patient. Thus, by being pre-loaded, the wires 40 may already extend out of the holes 34 and will not need to be routed through the generally small holes 34 of the balloon 18 after the balloon 18 is exposed from the sheath 14 and delivered to the desired delivery area. Accordingly, the wires 40 being pre-loaded will result in the wires 40 extending through the holes 34 prior to the balloon 18 being inflated.

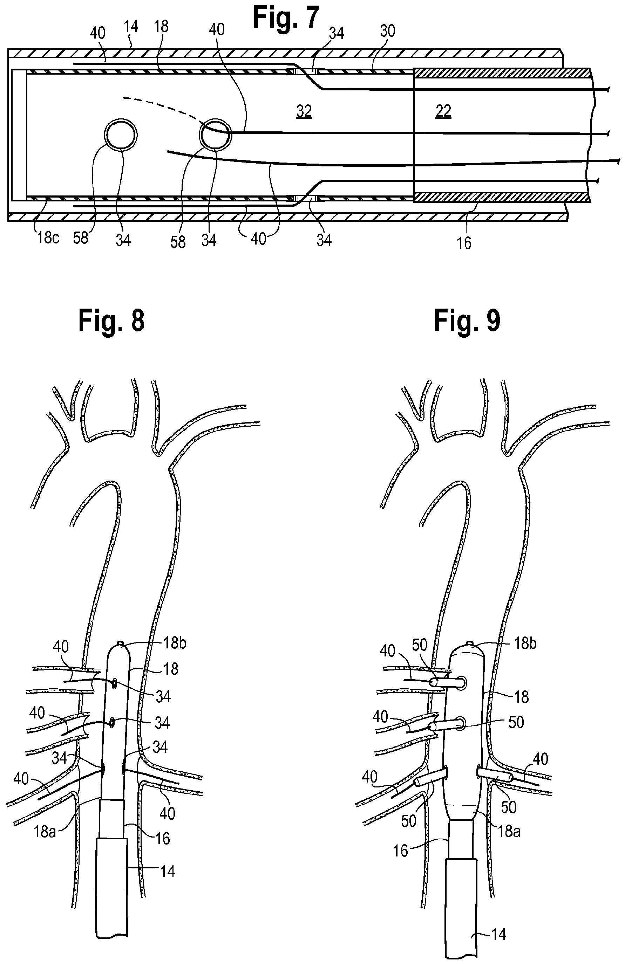

In another approach, the pre-loaded wires 40 may terminate within the balloon cavity 32 when the balloon catheter 12 is delivered in the delivery configuration, and the wires 40 may be carefully routed through the holes 34 after the balloon 18 has been exposed within the body lumen. In this approach, the balloon 18 may be partially inflated to increase the size of the cavity 32 to aid in routing the wires 40. In another approach, the wires 40 may not be pre-loaded and may be introduced through the balloon catheter 12 and into and through the cavity 32 after the balloon 18 has been delivered. Figure 7 illustrates wires 40 both extending through the holes 34 in the preloaded state and terminating within the cavity 32 in the preloaded state.

Thus, with the wires 40 extending from the delivery lumen 22 through the cavity 32 and out of the balloon 18 to the exterior of the balloon 18, additional components can be delivered along the wires 40 and will be routed to the corresponding hole 34 through which the wires 40 extend.

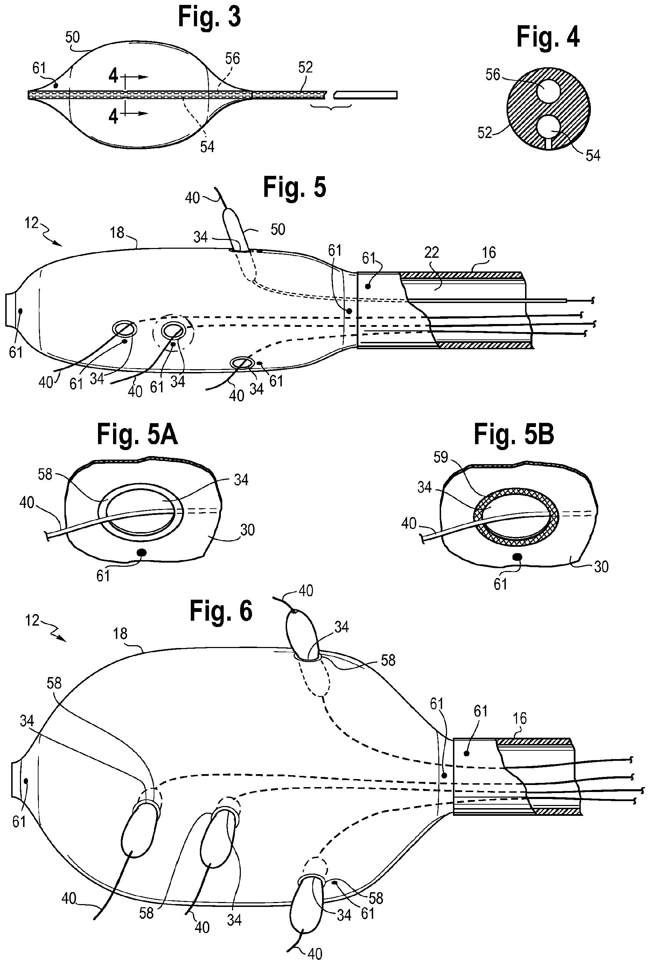

With reference now to Figures 3-6, the system 10 further includes one or more "additional," "secondary," or "daughter" balloons 50 that are configured to be delivered through the delivery lumen 22 over the wires 40 and into engagement with the holes 34 defined by the balloon 18.

Figures 3 and 4 shown examples of daughter balloons. The daughter balloons 50 may be attached to a shaft 52 having an inflation lumen 54 and a wire lumen 56 in the manner of a traditional balloon catheter. The inflation lumen 54 provides inflation fluid to the interior of the balloon 50 to inflate and expand the balloon 50 in a manner known in the art, and the balloon 50 and shaft 52 are deliverable over a wire via the wire lumen 56 in a known manner as well. Figure 4 illustrates one example of a lumen arrangement, showing the shaft 52 in cross-section. However, it will be appreciated that other arrangements of the lumens could be used, such as a dual lumen design for the inflation lumen 54, or a coaxial lumen design where the wire lumen 56 is disposed coaxially within the inflation lumen 54.

Thus, with reference to Figure 5, in one approach, individual daughter balloons 50 are deliverable over individual wires 40 through the delivery lumen 22 of the shaft 16 and into engagement with the hole 34 that corresponds to the wire 40 over which the particular individual balloon 50 was delivered. Figure 5 illustrates a first daughter balloon 50 being delivered to one of the holes 34 over the wire 40 that extends through the hole 34. Figure 5 further illustrates the other holes 34 having wires 40 extending therethrough. Figure 5 illustrates the balloon 18 in a partially inflated state, however delivery of the daughter balloons 50 may occur when the balloon 18 is still in a compressed state and prior to any inflation. It may be difficult to inflate the balloon 18 effectively prior to delivery of each of the daughter balloons 50 due to the holes 34 being present in the balloon 18.

The daughter balloons 50 may be made from traditional medical balloon materials, and can be compliant or semi-compliant, depending on the needs of the user. The size of the daughter balloons 50 can also be selected to correspond to a patient's particular anatomy. As described above with respect to the holes 34, the expanded width of the balloon 50 is preferably greater than the size of the hole 34, such that when the balloon 50 is expanded into engagement with the hole 34, the edge of the hole 34 will seal against the outer wall of the daughter balloon 50. The compliant nature of the balloon 18 will allow the hole 34 to stretch to accommodate the expanded outer width of the daughter balloon 18. In one approach, the daughter balloons 50 are more rigid than the balloon 18, such that the daughter balloons 50 will stretch the holes 34 in the balloon 18, as shown in Figure 5. In another approach, the daughter balloons 50 may be less rigid, and in this case the holes 34 may not stretch, and instead the daughter balloons 50 would expand further on each side of the hole 34 than in the hole 34, such that the diameter of the daughter balloon 50 on either side of the hole 34 is greater than the diameter of the hole 34, thus taking on a somewhat hourglass shape, as shown in Figure 6.

The balloons 50 are preferably delivered over the wires 40 in a sequential manner, such that the delivery lumen 22 of the shaft 16 can be sized to accommodate the number of preloaded wires 40 in addition to allowing a single balloon 50 to be delivered through the lumen 22, which keeps the overall width of the shaft 16 small. However, it would also be possible to increase the size of the delivery lumen 22 to allow for delivery of more than one balloon 50 at a time side-by-side, but this would also increase the width of the shaft 16 to a size larger than one where balloons 50 are delivered sequentially.

Figure 6 illustrates the balloon 18 in a fully inflated state after each of the daughter balloons 50 have been delivered to the corresponding holes 34 over the corresponding wires 40. The balloons 50 have been inflated and have created a seal with the holes 34, thereby sealing off the cavity 32 inside the balloon 18 such that the balloon 18 may be inflated. Figure 6 illustrates the example of the daughter balloons 50 taking on an hourglass shape after inflation, as described above.

Due to the expansion of the balloons 50 while extending through the holes 34 to create the seal, the balloon 34 may also include the reinforcing band 58 disposed around the edge of the holes 34, as shown in Figure 5A. The reinforcing band 58 can help prevent tearing of the balloon 18 at the location of the holes 34 when the holes 34 are stretched, and can also help provide a seal against the expanded daughter balloon 50. As shown in Figure 5A, the reinforcing band 58 can be in the form of an additional layer of balloon material that is bonded or adhered to the area surrounding the hole, or it could be in the form of an applied coating or curing adhesive. In another approach, as shown in Figure 5B, a mesh material 59 may be embedded in the wall 30 of the balloon 18 around the holes 34.

When the daughter balloons 50 are expanded into a sealing engagement with the holes 34 of the balloon 18, the balloon 18 will be generally sealed from inflation fluid leaking out of the balloon 18 when the balloon 18 is inflated. It has been found that the use of 8x20 mm sized daughter balloons 50 inserted into 2-4 mm holes 34 in the balloon 18 allows the balloon 18 to hold greater than 1 atm of pressure when inflation fluid is introduced into the cavity 32 to inflate the balloon 32. As shown in Figure 5, the balloon 18 and the holes 34 thereof, the daughter balloons 50, and shaft 16, as well as other structure described above, can each include radiopaque markers 61 disposed at various selected locations to aid in locating the balloon 18 and the various corresponding and cooperating structure at the desired location within the patient's anatomy. For example, markers 61 may be located at each of the holes 34 to assist in positioning the balloon 18 so that the wires 40 and daughter balloons 50 may be routed to the desired branch vessels. Similarly, the wires 40 may be made of a radiopaque material.

The daughter balloons 50 can be used to cannulate various branch vessels adjacent the delivered location of the main balloon 18. This can be performed quickly and easily due to the pre-loaded wires 40 that extend through the balloon 18 in its delivery state. The wires 40 are moveable relative to the shaft 16 and the holes 34 of the balloon 18, such that after the balloon 18 and wires 40 have been delivered, the wires 40 can be individually extended into the desired branch vessel prior to delivering the daughter balloon 50. Accordingly, the daughter balloon 50 will enter the desired branch vessel along the wire 40.

The system 10 has a delivery state and a deployed state. With reference to Figure 7, in the delivery state, the balloon 18 and shaft 16 and wires 40 are disposed within the delivery sheath 14 and covered by the delivery sheath 14. The wires 40 are pre-loaded in the balloon 18. Figure 7 shows three wires 40 extending through the holes 34 in the balloon 34 such that they extend through the holes 34 and to the exterior of the balloon 18 while in the delivery state. Figure 7 also illustrates one wire 40 terminating within the balloon cavity 32. It will be appreciated that all of the wires 40 may extend out of the holes 34 in the delivery state, all of the wires 40 may terminate with the cavity 32 in the delivery state, or some of the wires 40 may extend out of the holes 34 and others of the wires 40 may terminate within the balloon cavity 32.

In the deployed state, shown in Figure 8, the delivery sheath 14 is retracted relative to the balloon 18, shaft 16, and wires 40, thereby exposing the balloon 18 and the wires 14 to the surrounding vasculature. From this deployed state, the wires 40 may be routed into the desired branch vessels, and the balloons 50 may be introduced as described above and the balloon 18 may be inflated.

As shown in Figure 8, upon the balloon 18 being exposed by retracting the sheath 14 at the desired location, the wires 40 are routed into the desired adjacent branch vessels. As shown in Figure 9, following positioning of the wires 40, the daughter balloons 50 are delivered over the wires 40 and into the branch vessels while also being disposed within the holes 34 of the balloon 18. The balloons 50 are preferably delivered sequentially. However, as described above, in another approach multiple balloons 50 may be delivered side-by-side or otherwise together if the delivery lumen 22 is wide enough to accommodate multiple daughter balloons 50 in that arrangement.

After delivering the daughter balloons 50 over the wires 40 and into the holes 34 of the balloon 18, the daughter balloons 50 are inflated. The daughter balloons 50 can be inflated sequentially after delivering each individual balloon 50, or multiple balloons 50 may be inflated at the same time after delivering multiple balloons 50. In another approach, the balloons 50 could each be inflated at the same time after some or all of the balloons 50 have been delivered.

As the balloons 50 are inflated, they can be inflated to provide support to the branch vessels. In another approach, the balloons 50 may be positioned at different areas outside of the balloon 18 outside of a branch vessel. For example, one or more of the balloons 50 could be positioned against the main vessel wall.

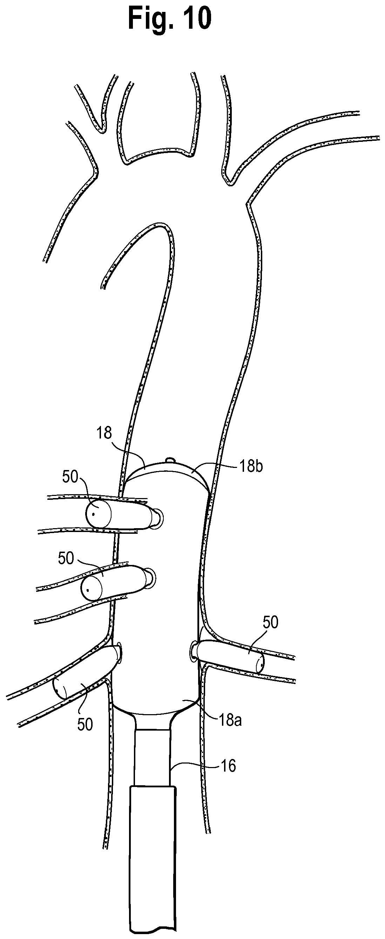

As shown in Figure 10, after each of the daughter balloons 50 have been inflated, the main balloon 18 is inflated and expanded into engagement with the main vessel wall. The previous inflation of the daughter balloons 50 provides a sealing and filling of the holes 34, such that the main balloon 18 will sufficiently inflate.

In one approach, the balloon 18 is compliant, and the daughter balloons 50 are minimally compliant, meaning that the daughter balloons 50 can inflate to a predefined shape, causing the balloon 18 to stretch in response to inflation of the daughter balloons 50. Therefore, when the balloon 18 is inflated, it will tend to conform to the shape of the vessel wall as well as around the daughter balloons 50. In another approach, the daughter balloons 18 may be compliant and will take the shape of the vessel in which they are deployed, and inflation of the balloon 18 may alter the shape of the daughter balloons 50, depending on whether the balloon 18 or daughter balloons 50 are more rigid relative to each other.

The main balloon 18 can be cycled between an inflated and deflated position to open up a blocked blood vessel. In this case, the daughter balloons 50 provide support to the branch vessels as the main balloon 18 is inflated and deflated. While the balloon 18 has been described as being compliant and conforming to the shape of the vessel, it will be appreciated that the balloon 18 may still be arranged to have sufficient rigidity when inflated to open up a blocked vessel.

In the case of aortic dissection, such as type B dissection, the daughter balloons 50 in their inflated state provide support to the branch vessels, while the inflation of the main balloon 18 provides support within the true lumen, thereby allowing for filling the false lumen with embolic material.

It will be appreciated that the above arrangement may be used in various other situations where balloon support for a main vessel and branch vessels is desired.

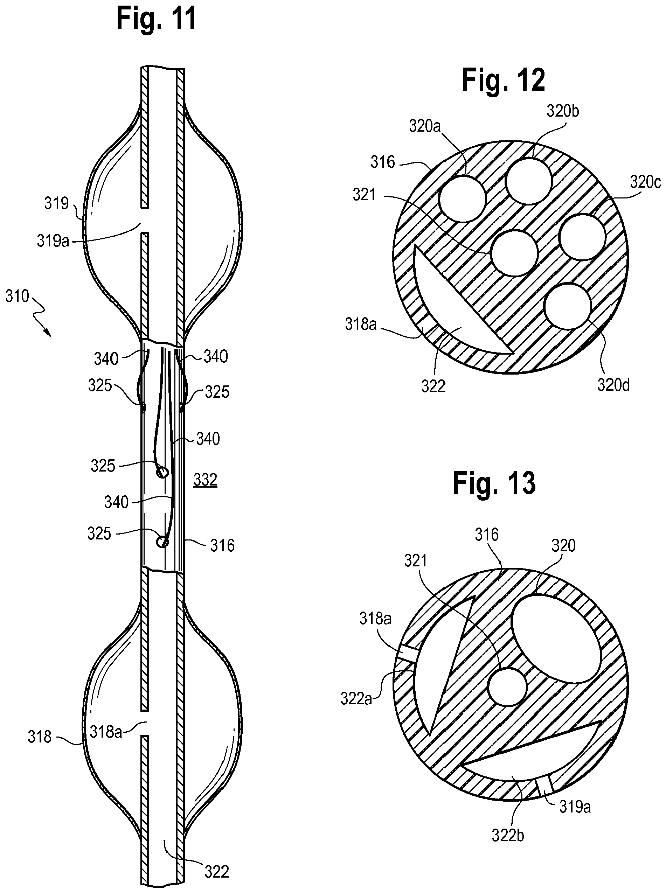

With reference to Figures 11-16, in another embodiment, a system 310 includes a shaft 316 that supports a first balloon 318 and a second balloon 319, each of which are attached to the shaft 316 in a manner known in the art for inflatable balloon catheters. The first balloon 318 is longitudinally spaced away from the second balloon 319, such that the balloons 318 and 319 are located at different longitudinal locations on the shaft 316. The balloons 318 therefore define an intermediate space 332 longitudinally between them when they are inflated, where the intermediate space 332 is located outside of the shaft 316.

The balloons 318 and 319 are configured to not include any holes or other openings in the outer wall of the balloon, similar to a traditional balloon catheter, and can be further configured to be used without defining any holes or other punctures by wires. Of course, it will be appreciated that the above described embodiments of the system 10 could be applied to the balloons 318 and 319, if desired.

The system 310 provides occlusion and/or support for varying or complex anatomy via the use of secondary balloons 350 that are smaller than the balloons 318 and 319, where the secondary balloons 350 are disposed longitudinally between the balloons 318 and 319 in the intermediate space 332 between them.

The balloons 318 and 319 are compliant or semi-compliant balloons, such that they will tend to conform to the shape of the vessel in which they are disposed and will support the vessel wall without damaging it. The secondary balloons 350 may be minimally compliant balloons. The secondary balloons 350 may be used to support various branch vessels, while the balloons 318 and 319 can be used to occlude a main vessel. The secondary balloons 350 may also be used to separate the balloons 318, 319 from the vessel wall at the location of the balloon 350, if desired to create a fluid passage along the side of the balloons 318, 319.

The shaft 316 includes, in one approach, a common inflation lumen 322 for providing inflation fluid to both the first balloon 318 and the second balloon 319. The inflation lumen 322 may be skived or pared or otherwise opened in different longitudinal locations along the shaft 316 that corresponds to the location of each of the balloons 318 and 319, thereby defining side-ports 318a and 319a, respectively, such that the inflation lumen 322 is in fluid communication with the interiors of the balloons 318 and 319 in a manner known in the art. Figure 12 illustrates one example of a lumen layout through the shaft 316 corresponding to the lumens described above.

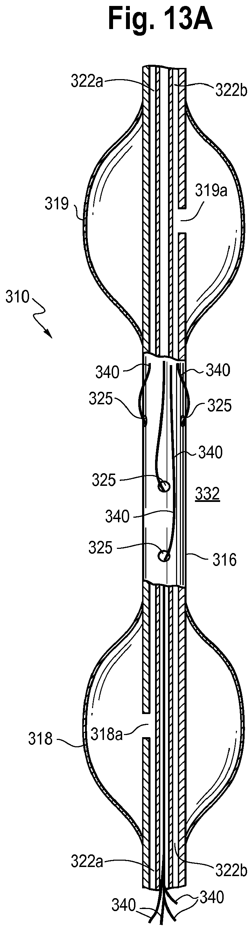

In another approach, inflation for each of the balloons 318 and 319 can be provided by dedicated inflation lumens 322a and 322b extending through the shaft 316. Figures 13 and 13A illustrates one example of a lumen layout having two inflation lumens 322a and 322b. In this approach, one of the inflation lumens 322a, 322b would be in fluid communication with side-port 318a, and the other would be in fluid communication with the side-port 319a.

In the case of the common inflation lumen 322, provision of inflation fluid into the lumen 322 will tend to inflate the balloons 318 and 319 at approximately the same time. In the case of the dedicated lumens 322a and 322b, the user can selectively inflate or deflate the balloons 318 and 319 separately, allowing for staggered inflation or simultaneous inflation, depending on the needs of the user. In some instances, it may be desirable for the user to inflate both balloons at the same time, while in other cases selected inflation and deflation of each balloon may be beneficial.

In addition to the inflation lumen 322 or lumens 322a, 322b, the shaft 316 further defines at least one delivery lumen 320 for delivering one or more wires 340 that are used for delivering the secondary balloon 350 to a desired location. In one approach, a single delivery lumen 320 can be used, such as that shown in Figure 13. The delivery lumen 320 can be used to carry one or more wires 340 over which the secondary balloons 350 will be delivered through the delivery lumen.

The shaft 316 may further include a guidewire lumen 321 allowing the shaft 316 to be delivered over a guidewire for delivery of the shaft 316 and the balloons 318 and 319 to the desired location within the vasculature.

The shaft 316 includes one or more holes or openings 325 that provides communication from within the lumen 320 to the outside of the shaft 316. The openings 325 may be spaced along the shaft 316 at different locations, allowing for the delivery of the secondary balloons 350 through the openings 325 to the corresponding area of the vasculature. The wires 340 that are used to deliver the secondary balloons 350 can be extended out of the desired opening 325 for delivery of the balloon 350 to the corresponding location. The wires 340 can also be preloaded through the various openings 325 such that they are delivered along with the shaft 316 and will already extend out of the openings 325 when the shaft 316 is delivered to the body. When the wires 340 are preloaded through the delivery lumen 320 and out of the holes 325, the wires 340 may be detachably attached to the shaft 316 outside of the shaft 316. Alternatively, the wires 340 may be deployed through the delivery lumen 320 after the shaft 316 has been delivered and deployed, and in this configuration the wires 340 would not be preloaded. The wires 340 may be preloaded by the physician prior to delivering the shaft 316, or the system may be manufactured and delivered to a physician with the wires 340 being preloaded.

In another approach, multiple delivery lumens 320a, 320b, 320c, etc. may be used for the delivery of wires 340 and secondary balloons 350 to the desired location. The multiple delivery lumens are illustrated in Figure 12. It will be appreciated that the multiple delivery lumens 320a, b, c, and d could replace the delivery lumen 320 in Figure 13, and vice-versa.

In this approach, the wires 340 will extend through the lumens 320a, 320b, etc. to the location defined by the openings 325 through the wall of the shaft 316 that are disposed at the end of the corresponding lumens. Thus, an individual opening 325 will correspond to the one of the multiple delivery lumens 320a, b, c, and d, rather than each of the openings 325 corresponding to the single delivery lumen 320.

This approach can make it easier to route the wires 340 through the lumen to the desired location, because a particular opening need not be selected when extending the wire 340. In the case of multiple delivery lumens, the guidewire lumen 321 may extend fully through the shaft 316 and be used for delivering the shaft 316 over a guidewire to the desired location within the vasculature.

While the balloons 318 and 319 are disposed at different longitudinal locations on the shaft 316, the balloons 318 and 319 can nevertheless be disposed relatively close to each other, such that inflation of the two balloons 318 and 319 will take up and fill the longitudinal space between them when inflated.

This can be effected by the body of the balloon 318/319 extending longitudinally beyond the bonding interface to the shaft 316. Additionally or alternatively, the balloon 318/319 can be sized such that increased inflation will push the body of balloon 318/319 into the open space longitudinally between them after the balloon has radially filled the vessel.

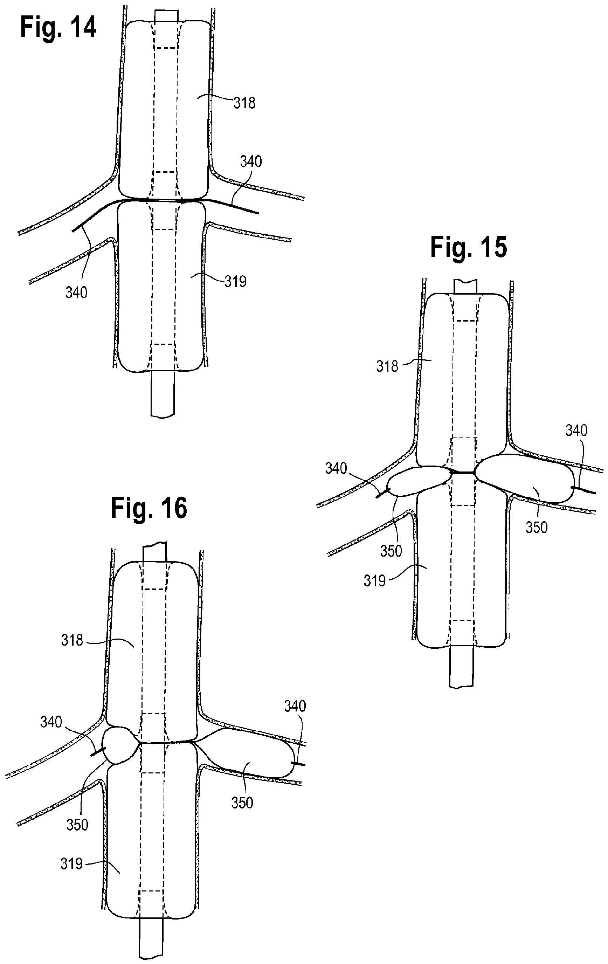

Figure 14 illustrates one example of the balloons 318 and 319 being inflated to fill the intermediate space. For clarity, some space is shown between the balloons 318 and 319 to illustrate the wires 340 extending between the balloons 318, 319, but it will be appreciated that the balloons 318 and 319 will engage other in the intermediate space when inflated even in the presence of the wires 340 due to the compliant nature of the balloons 318, 319. The filling of the intermediate space 332 between the balloons 318/319 when inflated of course excludes the space taken up by wires 340 or balloons 350 that are disposed within that space prior to inflation of the balloons 318/319.

In the case where wires 350 are not preloaded in the system 310 and are delivered later, the balloons 318, 319 are preferably not inflated into the intermediate space until the wires 340 have been routed to the desired location outside of the shaft 316. This approach can aid in preventing undesirable puncturing of the balloons 318 and 319 by the movement of the wires 350.

In using the system 310, occlusion of the vessel can be accomplished quickly and easily by inflating the two balloons 318 and 319. The occlusion can be selectively decreased by deflating the balloons 318 and 319 at least partially to allow for the introduction of the secondary balloons 350 into the intermediate space 332, if necessary. The occlusion can then be increased again to achieve a more complete seal of the vessel.

If the wires 340 are preloaded through the openings 325 such that they already extend into the intermediate space outside the shaft 316, or if the wires 340 are extended out of the shaft 316 into the intermediate space prior to inflating the balloons 318 and 319, the secondary balloons 350 can be delivered to the intermediate space or other desired location outside of the shaft 316 even when the balloons 318 and 319 are fully inflated.

Figure 15 illustrates an example of the balloons 350 being delivered between the inflated balloons 318, 319. The insertion of the balloons 350 into the space occupied by the compliant balloons 318 and 319 is possible because of the compliant nature of the balloons 318 and 319 and the minimally compliant nature of the secondary balloons 350. Further, because the wires 340 are already in place, potential damage to the compliant balloons 318 and 319 due to wire puncture is limited, and inserting the secondary balloons 350 over the wires 340 is less concerning.

With the secondary balloons 350 delivered, they may be expanded or inflated as known in the art. The secondary balloons 350 can be inflated across the compliant balloons 318/319, partially across the compliant balloons 318/319, or fully outside of and beyond the compliant balloons, as desired.

Figure 15 illustrates the secondary balloons 350 inflated partially across the balloons 318, 319. Figure 16 illustrates one secondary balloon 350 fully outside of the balloons 318,319, and another secondary balloon 350 across the balloons 318, 319.

The above described arrangement allows for the introduction of secondary balloons 350 to vary the occlusion and the quantity of included secondary balloons 350 can also be adjusted without affecting the integrity of the balloons 318 and 319.

However, as described above, the balloons 318 and 319 could also be arranged to include holes and daughter balloons similar to previously described embodiments, in addition to the inclusion of secondary balloons 350 being disposed in the intermediate space between the balloons 318 and 319. Alternatively, the longitudinally spaced balloons 318 and 319 could be used with daughter balloons without the inclusion of secondary balloons into the intermediate space, if desired.

Figures 14-16 illustrate two secondary balloons 350 being delivered between the balloons, but it will be appreciated that the system 310 can include multiple side-ports in the shaft to accommodate multiple wires 340 and secondary balloons 350 depending on the needs of the patient.

The method for delivering the system 310 is similar to the method described above with respect to the system 10. The shaft 316 and balloons 318 and 319 are delivered to the body vessel in a compressed delivery configuration. The balloons 318 and 319 are exposed from a delivery sheath. With the balloons 318, 319 exposed, the wires 340 are preferably routed to the desired location where the secondary balloons 350 are intended to be delivered. In one approach, the wires 340 are routed into adjacent branch vessels.

With the wires 340 in place, the balloons 318 and 319 may be inflated to take up the intermediate space 332. After inflating the balloons 318 and 319, the secondary balloons 350 may be delivered through the delivery lumen 320 (or through individual delivery lumens 320a, b, c, etc.). The compliant nature of the balloons 318, 319 will allow the secondary balloons 350 to move through the space occupied by the compliant balloons 318 and 318 if they are fully inflated. If the balloons 318 and 319 are partially inflated, the secondary balloons 350 will move through the intermediate space left between them. The balloons 350 may then be left in their desired location, and dilation and/or support of the body vessel is achieved.

In another approach, the secondary balloons 350 may be delivered over the wires 340 to the desired location prior to inflation of the balloons 318 and 319. With the secondary balloons 350 in their desired location, the balloons 318 and 319 may be inflated to take up the remaining space in the intermediate space 350.

Figure 14 illustrates the wires 340 in a desired location extending into adjacent branch vessels. Figure 15 illustrates the balloons in the desired location extending into adjacent branch vessels, with the balloons 318 and 319 inflated to take up the remaining intermediate space. The balloons 350 in Figure 15 may be delivered before or after the inflation of the balloons 318 and 319. Figure 16 illustrates a different desired location of the balloons 350, and these positions are also achievable either before or after the balloons 318 and 319 are inflated.

Throughout this specification various indications have been given as to preferred and alternative examples and aspects of the invention. However, the foregoing detailed description is to be regarded as illustrative rather than limiting and the invention is not limited to any one of the provided aspects. It should be understood that it is the appended claims, including all equivalents, that are intended to define the scope of this invention.

The disclosures in United States patent application number US-15/631,797, from which this application claims priority, and in the abstract accompanying this application are incorporated herein by reference.

## Claims

1. A medical system comprising:
a balloon catheter including a shaft and a first balloon attached to the shaft and a second balloon attached to the shaft, wherein the first and second balloons are spaced apart longitudinally on the shaft;
at least one inflation lumen extending within the shaft and in fluid communication with an interior cavity of the first balloon and an interior cavity of the second balloon;
wherein the first balloon and the second balloon have an intermediate space longitudinally between the first balloon and the second balloon outside of the shaft;
at least one delivery lumen extending longitudinally through the shaft and in fluid communication with the intermediate space via a side-port of the shaft, the delivery lumen configured to deliver medical devices through the shaft and into the intermediate space through the side-port; and
at least one secondary balloon moveable through the at least one delivery lumen and out of the side-port into the intermediate space;
wherein the first and second balloons are inflatable longitudinally toward each other to take up the intermediate space.

2. A system according to claim 1, wherein the at least one inflation lumen is a first inflation lumen and a second inflation lumen, and the first inflation lumen is in fluid communication with the interior cavity of the first balloon, and the second inflation lumen is in fluid communication with the interior cavity of the second balloon, wherein inflation of the first balloon and the second balloon is independently controllable.

3. A system according to claim 1, wherein the at least one inflation lumen is a single inflation lumen and is in fluid communication with both the interior cavity of the first balloon and the interior cavity of the second balloon, wherein inflation of both balloons is controllable via the same inflation lumen.

4. A system according to any preceding claim, wherein the at least one delivery lumen comprises multiple delivery lumens having corresponding side-ports, wherein each individual delivery lumen is in fluid communication with the corresponding side-port.

5. A system according to claim 4, wherein individual ones of the corresponding side-ports are disposed at different longitudinal or circumferential locations on the shaft.

6. A system according to claim 4 or 5, wherein each of the side-ports are disposed longitudinally between the first balloon and the second balloon.

7. A system according to any preceding claim, further comprising a wire extending through the at least one delivery lumen and out of the side port.

8. A medical system comprising:
a balloon catheter including a shaft and a first balloon attached to the shaft and a second balloon attached to the shaft, wherein the first and second balloons are spaced apart longitudinally on the shaft;
at least one inflation lumen extending within the shaft and in fluid communication with an interior cavity of the first balloon and an interior cavity of the second balloon;
wherein the first balloon and the second balloon have an intermediate space longitudinally between the first balloon and the second balloon outside of the shaft;
at least one delivery lumen extending longitudinally through the shaft and in fluid communication with the intermediate space via a side-port of the shaft, the delivery lumen configured to deliver medical devices through the shaft and into the intermediate space through the side-port;
at least one secondary balloon moveable through the at least one delivery lumen and out of the side-port into the intermediate space; and
at least one wire extending through the at least one delivery lumen, wherein the at least one secondary balloon is deliverable over the at least one wire;
wherein the system includes a delivery configuration in which the first and second balloons are in a compressed configuration, and the system includes a deployed configuration, wherein the first and second balloons are expanded; and
wherein the secondary balloons do not extend beyond the side-port in the delivery configuration, and the secondary balloons are disposed at least partially through the side-port in the deployed configuration.

9. A system according to claim 8, wherein, in the delivery configuration, the at least one secondary balloon is outside of the shaft.

10. A system according to claim 8 or 9, wherein, in the deployed configuration in which the first and second balloons are inflated, and the first and second balloons expand longitudinally toward each other and occupy the intermediate space.

11. A system according to claim 10, wherein, in the deployed configuration, the at least one secondary balloon is disposed in the intermediate space.

12. A system according to any one of claims 8 to 11, wherein the at least one wire extends through the at least one delivery lumen and out of the side-port into the intermediate space in the delivery configuration.

13. A system according to any one of claims 8 to 12, wherein the wire extends through the at least one delivery lumen and terminates prior to the side-port in the delivery configuration.

14. A system according to any one of claims 8 to 13, wherein the first and second balloons are compliant balloons, and the secondary balloon is minimally compliant, wherein the first and second balloons will conform to the shape of the body vessel and the shape of the secondary balloon when inflated.
